Europäisches Patentamt

(19) European Patent Office

Office européen des brevets

(11) Publication number: **0 070 685**
**B1**

(12) **EUROPEAN PATENT SPECIFICATION**

(45) Date of publication of patent specification: **16.10.85**

(21) Application number: **82303699.1**

(22) Date of filing: **14.07.82**

(51) Int. Cl.⁴: **G 01 N 33/58**

(54) **Homogeneous nucleic acid hybridization diagnostics by non-radiative energy transfer.**

(30) Priority: **17.07.81 US 284469**

(43) Date of publication of application:
**26.01.83 Bulletin 83/04**

(45) Publication of the grant of the patent:
**16.10.85 Bulletin 85/42**

(84) Designated Contracting States:
**BE CH DE FR GB IT LI NL**

(56) References cited:
**GB-A-2 018 424**
**US-A-4 174 384**
**US-A-4 302 204**

**CHEMICAL ABSTRACTS, vol. 81, no. 21,**
**November 25, 1974, abstract no. 131748v, page**
**85, Columbus Ohio (US), YANG, CHIH-HSIN et**
**al.: "Transfer RNA tertiary structure by singlet-**
**singlet energy transfer"**
**(THE WELSH NATIONAL SCHOOL OF**
**MEDICINE)**

(73) Proprietor: **AMOCO CORPORATION**
**200 East Randolph Drive**
**Chicago Illinois 60601 (US)**

(72) Inventor: **Heller, Michael James**
**30 W 057-104 Granada**
**Naperville Illinois 60540 (US)**
Inventor: **Morrison, Larry Edward**
**4913 Spencer**
**Lisle Illinois 60532 (US)**
Inventor: **Prevatt, William Dudley**
**1205 Grant Street**
**Downers Grove Illinois 60515 (US)**
Inventor: **Akin, Cavit**
**1462 Inverrary Drive**
**Naperville Illinois 60540 (US)**

(74) Representative: **Ritter, Stephen David et al**
**Mathys & Squire 10 Fleet Street**
**London EC4Y 1AY (GB)**

Courier Press, Leamington Spa, England.

# Description

## Background of the invention

Presently, nucleic acid hybridization assays are used primarily in the field of molecular biology as a research tool for the detection and identification of a unique deoxyribonucleic acid (DNA) sequence or specific gene in a complete DNA, a mixture of DNA's, or mixture of DNA fragments. A number of variations of the technique exist and are used in recombinant DNA research. (See Methods in *Enzymology*, Vol. 68, R. Wu (Ed.) pp, 379—469, 1979; and Dunn, A. R., and Sambrook, J., *Methods in Enzymology*, Vol. 65; Part 1, pp. 468—478, 1980). One of the most widely used procedures is called the Southern blot filter hybridization method (Southern, E., J. Mol. Biol, *98*, 503, 1975). This procedure is usually used to identify a particular DNA fragment separated from a mixture of DNA fragments by electrophoresis. The procedure is generally carried out by isolating a sample of DNA from some organism. The isolated DNA is subjected to restriction endonuclease digestion and electrophoresed on a gel (agarose, acrylamide). When the gel containing the separated DNA fragments is put in contact (blotted) with a nitrocellulose filter sheet (or diazotized paper), the fragments are transferred and become bound to the nitrocellulose sheet. The gel-transfer nitrocellulose sheet containing the DNA fragments is then heated (~95°C) to denature the DNA. At this point the sheet is treated with a solution containing denatured radiolabeled (32p) "specific DNA probe" and hybridization is allowed to take place. Hybridization can take from 3—48 hours, depending on given conditions. Unhybridized "specific DNA probe" is then washed away. The nitrocellulose sheet is placed on a sheet of X-ray film and allowed to expose, which usually takes several days at −70°C. The X-ray film is then developed. The exposed areas on the film identify which DNA fragments have hybridized and therefore have a sequence similar to that of the "specific DNA probe". This procedure, as well as most other variations, requires the use of radioisotopes and is obviously very complex and time consuming. Because of these and other problems, DNA hybridization assays have remained only as a tool for basic research and have not been generally used in applied or commercial areas such as for clinical diagnostics.

By way of further background, the phenomenon of non-radiative energy transfer has also been utilized as an analytical tool. It occurs when one light emitting species is very close to another species which absorbs light energy within the emission spectrum of the emitting species. This energy transfer is most closely approximated by the Forster equation. (See "Energy Transfer and Organic Photochemistry" Vol. XIV, P. A. Leemakers and A. Weissberger, pp. 17—132, (1969), Interscience, New York). The use of the non-radiative energy concept has been described in a number of patents in connection with immunofluorescent assays. (See U.S. Patent Nos. 3,996,345; 3,998,943; 4,160,016, 4,174,384; and 4,199,559, all issued to E. F. Ullmann or Ullmann and M. Schwarzberg). These patents are closely related and generally pertain to assays wherein the fluorescent light emitted from an irradiated sample is diminished in the presence of a species (quencher) which absorbs some of the light energy. For a Similar discussion, see "Fluorescent Excitation Transfer Immunoassay", *The Journal of Biological Chemistry*, Vol. 251, No. 14, pp. 4172—4178 (July 25, 1976). Also see Fluorescamine and Fluorescein as Labels in Energy-Transfer Immunoassay", *Analyst*, Vol. 105, pp. 91—92 (January 1980). In addition, energy-transfer techniques have been used to determine the tertiary structure of transfer RNA's (C—H Yang and D. Sol PNAS, Vol. 71, No. 7, pp. 2838—2842, 1974).

There is a definite need in the area of clinical diagnostics for a simple and rapid method for detecting and identifying unique nucleotide (genome) sequences. For example, many so-called "slow infection" diseases of humans and animals where symptoms appear long after the infectious process is initiated are caused by virus or virus-like agents. Some of these diseases include Kuru, Kreutzfeldt-Jakob disease, subacute sclerosing panencephalitis, and progressive multifocal leukoencephalopathy. There is also evidence that more common human diseases, such as multiple sclerosis (MS) may be slow infections caused by measles virus. In many cases the viral agents believed to cause these slow infection diseases cannot be detected by immunodiagnostic techniques because no viral antigens are present. Therefore, hybridization assays are used to directly detect the viral genome (A. T. Haase, et al. Science, 212, pp. 672—674, 1981). Hybridization assays would also be useful in determining antibiotic resistance traits of many pathogenic microorganisms through detection of the resistance factor genome. Thus, hybridization diagnostics could play an important role in any case where low or no antigenic response precludes the use of immunodiagnostic techniques. However, for wide spread commercial use in clinical diagnostics, such a hybridization method should be relatively fast, simple to carry out, highly specific, highly sensitive, and if possible not involve the use of radioisotopes. Presently such a method is not available.

## Summary of the invention

In general, the invention relates to a homogeneous hybridization assay which is based on the inherent high fidelity of the base recognition process in double-stranded (ds) polynucleotides (DNA, RNA, DNA-RNA and synthetic polynucleotides) and the phenomenon of non-radiative energy transfer. It also relates to a hybridization system that does not involve the use of radioisotopes, but instead involves a chemiluminescent moiety and an absorber/

emitter moiety, which under proper conditions can provide sensitivity equal to that of radioisotopes. Most importantly it involves the use of two polynucleotide reagent strands in such a way that the hybridization assay is carried out in a homogeneous fashion. This means target polynucleotide sequences can be detected and identified in solution without the need to carry out any immobilization procedures. Also, because complete hybridization is necessary in order to produce the appropriate energy transfer generated light signal for detection, this method can be much more selective than any method presently available.

In one aspect, the invention resides in a diagnostic method for determining the presence of viruses, bacteria, and other microorganisms, as well as the existence of certain genetic expressions, by assaying for a particular single-stranded (ss) polynucleotide sequence which is characteristic of the target microorganism or genetic expression being assayed. In particular, the method comprises contacting the sample, under hybridization conditions, with first and second ss-polynucleotide reagent segments which are complementary to substantially mutually exclusive portions of the target ss-polynucleotide, said first reagent segment having a chemiluminescent moiety and said second reagent segment having an absorber/emitter moiety positioned such that, upon hybridization with a target ss-polynucleotide, the chemiluminescent moiety and the absorber/emitter moiety are close enough in proximity to permit non-radiative energy transfer (generally within about $10^{-8}$m (100Å) or less of each other); further contacting the sample with agents effective for inducing light emission from the chemiluminescent moiety; and measuring the quantity of light emitted by the absorber/emitter to determine amount of hybridization.

In a further aspect the invention resides in the foregoing method wherein the first ss-polynucleotide reagent segment also has an absorber/emitter moiety which absorbs a shorter wavelength of light than the absorber/emitter moiety on the second reagent segment, but emits light in a wavelength region that overlaps with the absorbance region of the absorber/emitter moiety on the second reagent segment. The hybridized sample is then irradiated with light of appropriate wavelength to excite the absorber/emitter moiety on the first reagent segment and the amount of hybridization is determined by measuring the quantity of light emitted from the absorber/emitter on the second reagent segment.

In a further aspect, the invention resides in the reagents useful in carrying the methods described.

The term "absorber/emitter moiety" as used herein refers to a species capable of absorbing light energy of one wavelength and emitting light energy of another wavelength. The term includes both phosphorescent and fluorescent species. In choosing the particular absorber/emitter for a given reagent system, it is necessary that it possess absorbance in the spectral region of the light produced by the chemiluminescent moiety (or the first absorber/emitter moiety, as the case may be). It is preferable that the emission of the absorber/emitter be of a long enough wavelength to be effectively distinguished from the chemiluminescence emitted by the reagent system.

For example, two chemiluminescent reactions of primary interest are luminol oxidation by hydrogen peroxide and aldehyde oxygenation (e.g. isobutyraldehyde and propanal). Both of these reactions are catalyzed by peroxidase. Suitable absorber/emitters for the luminol chemiluminescent reaction include free base porphyrins such as uroporphyrin and tetracarboxyphenylprophyrin, metalloporphyrins containing such metals as magnesium or zinc, tetraphenylcarboxyporphyrins, perylene, anthracene, 7-methyldibenzo (a,h) pyrene, and other polycyclic aromatics having conjugated ring systems of sufficient size to produce strong absorbance in the region of luminol chemiluminescence (between 400 and 450 nm). The absorber/emitters may be easily sulfonated and activated for conjugation by formation of the sulfonic acid chlorides by general synthetic procedures. Also, carboxylation may be performed if required. Suitable absorber/emitters for the chemiluminescence resulting from aldehyde oxygenation include the above-mentioned porphyrins and polynuclear aromatics. However, halogenation of the polynuclear aromatics is required in order to provide efficient transfer of energy from the chemiluminescent emitter since it emits from a triplet excited state. Examples of appropriate halogenated polynuclear aromatics are 9,10-dibromoanthracene, 9,10-dibromo-2,6-anthracene disulfonic acid, 3,10-dibromo-4,9-perylene dicarboxylate, and 3,9- or 3,10-dibromoperylene. If required, sulfonation or carboxylation as described are also easily performed on these compounds by general synthetic procedures.

In cases where both the first and second ss-polynucleotide reagent segments are to be labeled with absorber/emitter moieties, combinations of fluorescent compounds such as an etheno-nucleotide with a tetracarboxyperylene derivative or a fluorescein derivative with a rhodamine derivative can be used. Criteria for choosing absorber/emitter pairs are: (1) the absorber/emitter moiety on one reagent strand should have good absorption of light in the emission region of the absorber/emitter moiety on the second strand; (2) the final emission (fluorescence) should be strong and have a maximum sufficiently longer than that of the maximum of the first emission; and (3) both moieties should have properties which will allow them to be easily functionalized and coupled to the reagent strands.

The term "chemiluminescent moiety" includes any of a variety of moities which are capable of participating in light emitting reactions and which can be covalently attached to the ss-polynucleotide reagent segment. Such labels include those of both the chemiluminescent and bioluminescent types and as used herein the term "chemiluminescent" shall include the closely related term "bioluminescent". Chemiluminescent moieties useful within the scope of this invention include chemiluminescent catalysts such as peroxidase, bacterical luciferase and firefly luciferase and other chemiluminescent moities such as functionalized iron-porphyrin derivatives, and others. Choice of the chemiluminescent moiety depends on several factors, which include: (1) hybridization condtions to be used, particularly temperature; (2) method to be used for covalent coupling to the ss-polynucleotide reagent segment; and (3) size of the ss-polynucleotide reagent segment. The chemiluminescent reagents effective for inducing light emission from the chemiluminescent moieties will depend upon the particular chemiluminescent system being used and are well documented in the literature (*Methods in Enzymology*, Vol. LVII, M. A. Deluca (Ed.), 1978). For example, the following reaction illustrates how light is emitted in the presence of a peroxidase catalyst:

$$(1) \quad H_2O_2 + \text{Luminol} \xrightarrow{\text{Peroxidase}} \text{Oxyluminol} + H_2O + N_2 + h\nu$$

The chemiluminescent agents effective for inducing light emission in this instance would comprise hydrogen peroxide and luminol. Other agents which could be used include isobutyraldehyde and oxygen. Similar reagents are suggested by the following reactions using other chemiluminescent systems:

$$(2) \quad FMNH_2 + O_2 + RCHO \xrightarrow{\text{Bacterial Luciferase}} FMN + RCOOH + H_2 + h\nu$$

wherein $FMNH_2$ is reduced flavin mononucleotide, R is a straight carbon chain having from 8 to 12 carbons, and FMN is flavin mononucleotide.

$$(3) \quad \text{Luciferin} + ATP + O_2 \xrightarrow{\text{Firefly Luciferase}} \text{Oxyluciferin} + AMP + CO_2 + PPi + h\nu$$

wherein ATP is adenosine triphosphate, AMP is adenosine monophosphate, and PPi is inorganic phosphates.

The "target" ss-polynucleotide is a segment of either one of the two complementary strands of the double-stranded nucleic acid from the organism for which the assay is being conducted. It contains the unique polynucleotide sequence by which the organism itself or certain genetic traits can be identified.

The first and second ss-polynucleotide reagent segments must consist essentially of bases which are complementary to the base sequence of the target ss-polynucleotide. It is necessary that the first and second segments be complementary to substantially mutually exclusive portions of the target ss-polynucleotide. In other words, upon hybridization with the target polynucleotide the first and second reagent segments should not compete for the same base sequence to the extent that hybridization is prevented. That is, the first and second segments will line up head to tail (3' end to 5' end) with no overlap and with few or no base-pairing spaces left between them. First and second ss-polynucleotide reagent segments can be made from appropriate restriction endonuclease treated nucleic acid from the organism of interest or, in cases where the base sequence of a unique portion is known, they can be synthesized by organic synthetic techniques (Stawinski, J. et al., Nucl. Acids Res. 4, 353, 1977; Gough, G. R. et al., Nucl. Acids Res. 6, 1557, 1979;

Gough, G. R. et al., Nucl. Acids Res. 7, 1955, 1979; Narang, S. A., *Methods in Enzymology*, Vol. 65, Part I, 610—620, 1980). Also, it is possible to produce oligodeoxyribonucleotides of defined sequence using polynucleotide phosphorylase (E. Coli) under proper conditions (Gillam, S., and Smith, M., *Methods in Enzymology*, Vol. 65, Part I, pp, 687—701, 1980).

The first and second ss-polynucleotide reagent segments are generally labeled with · their appropriate moieties in the 3' terminal position and 5' terminal position respectively, that is, the 3' terminal position of one strand that will become continuous (line up head to tail) with the 5' terminal position of the other strand. Labeling of the 3' or 5' position with either chemiluminescent moiety or a given absorber/emitter moiety is arbitrary. In general it will depend on the given moiety and method of the coupling reaction.

The size of the reagent segments can be from 10 nucleotides to 100,000 nucleotides in length. Below 10 nucleotides, hybridized systems are not stable and will begin to denature above 20°C. Above 100,000 nucleotides, one finds that hybridization (renaturation) becomes a much slower and incomplete process, see (*Molecular Genetics*, Stent, G. S. and R. Calender, pp. 213—219, 1971). Ideally the reagnet segments should be from 20 to 10,000 nucleotides. Smaller nucleotide sequences (20—100) would lend themselves to production by automated organic synthetic techniques. Sequences from 100—10,000 nucleotides could be obtained from appropriate restriction endonuclease treatments. The labeling of the smaller segments with the relatively bulky chemiluminescent moieties may in some cases interfere with the hybridization process. In these cases it may be advantageous to use both reagent segments with appropriate absorber/emitter moieties.

The proper hybridization conditions will be determined by the nature of the light label attached to the reagent polynucleotide sequences, the size of the reagent polynucleotide sequences, the [G]+[C] (guanine plus cytosine) content of the reagent and sample polynucleotide sequences, and how the sample polynucleotide sequence is prepared. The light label can affect the temperature and salt concentration used for carrying out the hybridization reaction. Chemiluminescent catalysts can be sensitive to temperatures and salt concentrations that absorber/emitter moieties can tolerate. The size of the reagent polynucleotide sequences affects the temperature and time for the hybridization reaction. Assuming similar salt and reagent concentrations, hybridizations involving reagent polynucleotide sequences in the range of 10,000 to 100,000 nucleotides might require from 40 to 80 minutes to occur at 67°C, while hybridizations involving 20 to 100 nucleotides would require from 5 to 30 minutes at 25°C. Similarly, [G]+[C] content of the reagent and sample polynucleotide sequences affects the temperature and time for the hybridization reaction. Polynucleotide sequences with a high [G]+[C] content will hybridize at lower temperatures in a shorter period of time than polynucleotide sequences with a low [G]+[C] content. Finally, conditions used to prepare the sample polynucleotide sequence and maintain it in the single-stranded form can affect the temperature, time, and salt concentration used in the hybridization reaction. The conditions for preparing the sample polynucleotide sequence are affected by the polynucleotide length required and the [G]+[C] content. In general, the longer the sequence or the higher the [G]+[C] content, the higher the temperature and/or salt concentration required.

Brief description of the drawing

Figure 1 illustrates the preparation of first and second ss-polynucleotide reagent segments labeled with chemiluminescent catalyst and absorber/emitter moieties for use as reagents in carrying out an assay for antibiotic resistance.

Figure 2 illustrates the interaction between the sample and the first and second reagent ss-polynucleotide reagent segments, showing how the presence of the target ss-polynucleotide causes induced light emission (fluorescence).

Discussion

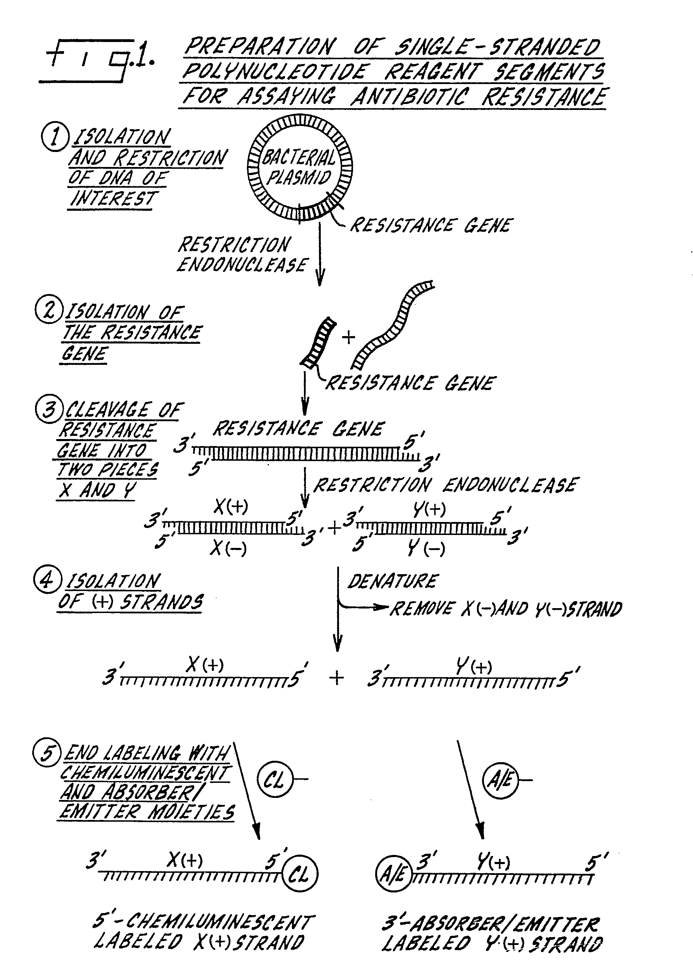

Directing attention to the Drawing, the invention will be described in greater detail. In Figure 1, the preparation of reagent ss-polynucleotides for assaying the presence of the antibiotic resistance gene, for example, is illustrated. Generally speaking, the most practical means to prepare the first and second reagent segments is to first isolate the particular polynucleotide containing the unique sequence of interest. In this case, for example, the antibiotic resistance gene located in a bacterial plasmid is obtained by subjecting the plasmid to the action of appropriate restriction enzymes. The gene of interest is separated from the other fragments by suitable methods, such as gel electrophoresis.

The isolated gene is then cut into two or more segments having contiguous ends by further action of an appropriate restriction enzyme. It is preferable to have only two segments of roughly equal size for matters of convenience and simplicity, but more than two segments can also be used equally well. The two gene segments have been labeled X and Y in the Drawing for purposes of identification. Also, each polynucleotide strand of each segment is labeled (+) or (−) for further identification. The two segments are denatured to liberate the four different ss-polynucleotide segments. At this point it is necessary to remove either the (+) segments or the (−) segments. Unless circumstances suggest otherwise, the choice is arbitrary. Figure 1 shows removal of the X(−) and Y(−) strands, leaving the X(+) and Y(+) strands, which represent first and second ss-polynucleotide segments which are complementary to mutually exclusive portions of the target ss-polynucleotide from which they were separated. (The base sequence of the original single-strand comprising the X(−) and Y(−) strands becomes the "target" ss-polynucleotide, whose presence in a ss-polynucleotide sample represents the presence of the antibiotic resistance (target) gene in the original physiological sample). Removal of the X(−) and Y(−) strands is most easily accomplished by exposing the strands to immobilized X(+) and Y(+) segments under hybridization conditions. Under such conditions the X(−) and Y(−) strands bond to the immobilized segments and can easily be removed from the system.

The contiguous ends of the remaining X(+) and Y(+) gene segments are then 5′-terminal and 3′-terminal labeled. The X(+) gene segment is 5′-terminal labeled with the chemiluminescent catalyst (CL) and the Y(+) gene segment is 3′-terminal labeled with an absorber/emitter moiety (A/E). These labeled strands become the first and second ss-polynucleotide reagent segments respectively. Upon hybridization, the chemiluminescent moiety and the absorber/emitter moiety will be positioned closely enough to permit efficient non-radiative energy transfer, generally within about $10^{-8}$m (100Å) or less of each other. In practice it may or may not be necessary to limit the labeling to the contiguous ends since the presence of extra labels at the other ends of the segment will not adversely affect the assay, unless they are within $10^{-8}$m

(100Å) or less of each other where energy-transfer can begin to take place. Therefore, for 22-polynucleotide reagent segments which are less than about 30 nucleotides ($\sim 9.10^{-9}$–$10^{-8}$m=$\sim$90—100Å) in length), only contiguous ends should be labeled. For segments which are more than 30 nucleotides ($\sim 10^{-8}$m=$\sim$100Å) in length, both ends can be labeled. In the latter case, all 5'-termini (X(+) and Y(+)) could be labeled with chemiluminescent catalysts and all 3'-termini (X(+) and Y(+)) could be absorber/emitter labeled. The decision to label 5'-terminal positions with chemiluminescent catalysts and 3'-terminal positions with absorber/emitter moieties, or visa versa, is arbitrary and determined by the functionality of the derivatives and coupling methods available.

As an example, the 3'-termini of the X(+) and Y(+) segments can be labeled with aminohexane-3'-5'-adenosine diphosphate using RNA ligase or with aminohexane-5'-adenosine diphosphate using polynucleotide phosphorylase under special conditions. The strands would contain an aminohexyl functional group at the 3'-termini through which a variety of absorber/emitter moieties could be easily attached. Coupling of a chemiluminescent catalyst, such as peroxidase, to the 5'-termini involves the synthesis of a short oligonucleotide linker segment. The segments contain an aminohexane-adenosine nucleotide at the 5'-termini followed by a short sequence of about four to six adenosine or thymidine nucleotides. This linker segment can now be attached to the 5'-terminal position of the X(+) and Y(+) segments through the appropriate use of basic ligation reactions, which are commonly used in recombinant DNA technology for plasmid construction.

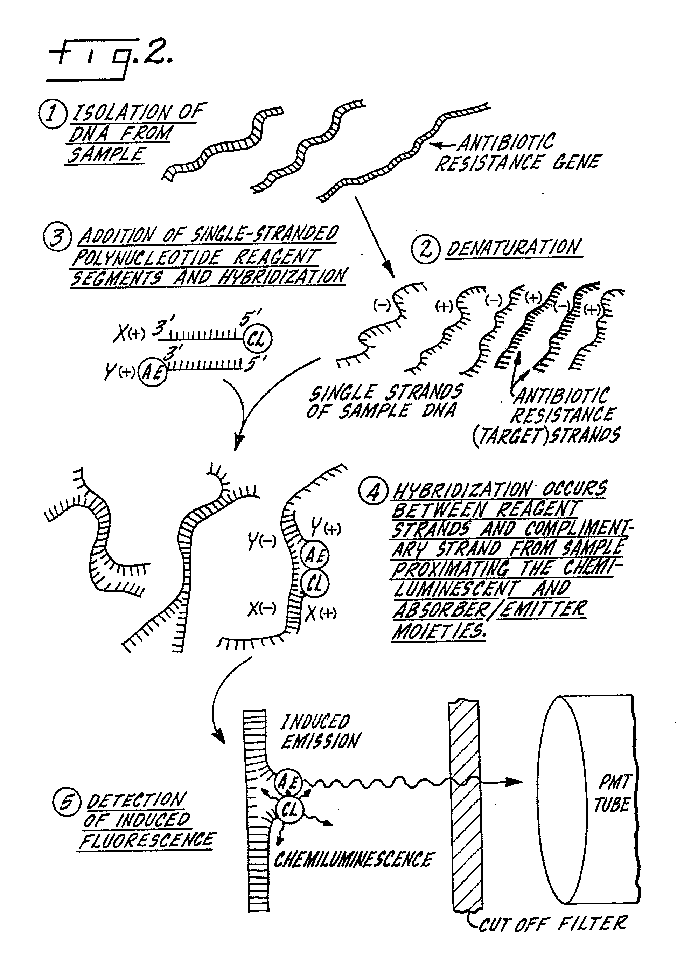

Figure 2 illustrates how the two ss-polynucleotide reagent segments interact with the sample ss-polynucleotides in performing the assay. Some sample preparation is necessary to free the DNA or RNA from its cells in the physiological sample. Preferably the polynucleotides from the original physiological sample are isolated to form a more concentrated sample. Sample DNA that has been isolated must be denatured to form a "single-stranded polynucleotide sample". This is the sample on which the assay of this invention is performed. Regardless of which strands ((+) or (−)) were used in preparing the first and second reagent segments, the ss-polynucleotide sample will contain the complementary strand if the gene being assayed was originally present. Figure 2 illustrates hybridization taking place when the ss-polynucleotide sample is contacted with the two reagent segments and the other chemiluminescent reagents. Such hybridization places the absorber/emitter in close proximity to the chemiluminescent catalyst such that non-radiative energy transfer can take place. This transfer of chemiluminescent energy excites the absorber/emitter and fluorescent light, for example, is emitted as shown. All light from the assay is preferably filtered to remove background chemiluminescence and is detected by a photomultiplier tube. For purposes of simplicity, it is preferable to have all of the reagents in one solution so that the only physical steps involved in the assay are to add the ss-polynucleotide sample and detect the light emitted, if any. However, it is also suitable to carry out the hybridization first, followed by further addition of the other chemiluminescent reagents necessary to create the chemiluminescent light response from the first ss-polynucleotide segment.

In cases where both the first and second ss-polynucleotide reagent segments are both absorber/emitter-labeled, the first absorber/emitter is irradiated with light at the appropriate wavelength and the second absorber/emitter emission wavelength is monitored for determining degree of hybridization.

**Claims**

1. A method for assaying a unique polynucleotide sequence or gene segment comprising:

a) contacting a single-stranded polynucleotide sample, under hybridization conditions, with first and second single-stranded polynucleotide reagent segments which are complementary to mutually exclusive portions of a target single-stranded polynucleotide, said first reagent segment having a chemiluminescent moiety attached thereto and said second reagent segment having an absorber/emitter moiety attached thereto, such that upon hybridization with a target single-stranded polynucleotide, the chemiluminescent moiety and the absorber/emitter moiety would be close enough to each other to permit non-radiative energy transfer;

b) further contacting the single-stranded polynucleotide sample with chemiluminescent reagents effective for causing light emission in the presence of the chemiluminescent moiety; and

c) measuring the quantity of light emitted by the absorber/emitter moiety.

2. A method according to Claim 1 wherein the single-stranded polynucleotide sample is contacted with the first and second polynucleotide reagent segments prior to being contacted with the chemiluminescent reagents.

3. A method according to Claim 1 or Claim 2 wherein the chemiluminescent moiety is selected from peroxidase and luciferases.

4. A method according to any preceding claim wherein the absorber/emitter moiety is selected from fluorophores and phosphores.

5. A method according to any preceding claim wherein the first and second single-stranded polynucleotide reagent segments are obtained by:

a) cleaving double-stranded target poly-nucleotides in a physiological sample to form polynucleotide segments;

b) denaturing the polynucleotide segments to obtain single-stranded polynucleotide segments;

c) end labeling the 3′ ends of the polynucleotide segments with either a chemiluminescent moiety or an absorber/emitter moiety; and

d) end labeling the 5′ ends of the polynucleotide segments with the moiety not used in step (c).

6. A method according to Claim 5 wherein the chemiluminescent moiety is attached to the 5′ ends of the polynucleotide reagent segments and the absorber/emitter moiety is attached to the 3′ ends of the polynucleotide reagent segments.

7. A method according to Claim 5 wherein the chemiluminescent moiety is attached to the 3′ ends of the polynucleotide segments and the absorber/emitter moiety is attached to the 5′ ends of the polynucleotide segments.

8. A reagent for detecting the presence of a target single-stranded polynucleotide in a single-stranded polynucleotide sample comprising:

a) a first single-stranded polynucleotide reagent segment having at least one chemiluminescent moiety attached thereto and which is complementary to a portion of a target single-stranded polynucleotide;

b) a second single-stranded polynucleotide reagent segment having at least one absorber/emitter moiety attached thereto and which is complementary to a different, mutually exclusive portion of the target single-stranded polynucleotide; and

c) chemiluminescent reagents effective for causing light emission in the presence of the chemiluminescent moiety.

9. A reagent according to Claim 8 wherein a chemiluminescent moiety is attached at an end of the first single-stranded polynucleotide reagent segment.

10. A reagent according to Claim 8 or Claim 9 wherein an absorber/emitter moiety is attached at an end of the second single-stranded polynucleotide reagent segment.

11. A reagent according to Claim 8 wherein the first single-stranded polynucleotide reagent segment contains a chemiluminescent moiety and an absorber/emitter moiety.

12. A reagent according to Claim 8 wherein both the first and second single-stranded polynucleotide reagent segments contain a chemiluminescent moiety and an absorber/emitter moiety.

13. A reagent according to Claim 12 wherein the chemiluminescent moiety and the absorber/emitter moiety are attached to the ends of both the first and second polynucleotide reagent segments.

14. A reagent according to Claim 13 wherein the chemiluminescent moiety is attached at the 3′ end of the first and second single-stranded polynucleotide reagent segments and an absorber/emitter moiety is attached at the 5′ end of the first and second single-stranded polynucleotide reagent segments.

15. A reagent according to any one of Claims 8 to 14 wherein the chemiluminescent moiety is selected from peroxidase and luciferases.

16. A reagent according to any one of Claims 8 to 14 wherein the absorber/emitter moiety is selected from fluorophores and phosphores.

17. A method for assaying a unique polynucleotide sequence or gene segment comprising:

a) contacting a single-stranded polynucleotide sample, under hybridization conditions, with a reagent solution comprising first and second single-stranded polynucleotide segments which are complementary to mutually exclusive portions of a target single-stranded polynucleotide, both of said first and second segments having a different absorber/emitter moiety attached thereto, such that upon hybridization with a target single-stranded polynucleotide the absorber/emitter moieties would be close enough to each other to permit non-radiative energy transfer;

b) irradiating the sample with light sufficient to excite one of the absorber/emitter moities; and

c) measuring the light response from the other absorber/emitter moiety.

18. A reagent solution comprising first and second single-stranded polynucleotide segments which are complementary to mutually exclusive portions of a target single-stranded polynucleotide, both of said first and second segments having a different absorber/emitter moiety attached thereto, such that upon hybridization with a target single-stranded polynucleotide the absorber/emitter moieties would be close enough to each other to permit non-radiative energy transfer.

**Revendications**

1. Un procédé pour l'analyse d'une séquence de polynucléotides particulière ou d'un segment de gène particulier comprenant:

a) le contact d'un échantillon de polynucléotide à brin unique dans des conditions d'hybridation avec un premier et un second segments réactifs de polynucléotides à brin unique qui sont complémentaires de portions s'excluant mutuellement d'un polynucléotide à brin unique cible, ledit premier segment réactif ayant un fragment chimioluminescent qui lui est attaché et ledit second segment réactif ayunt un fragment absorbeur/émetteur qui lui est attaché, si bien que lors de l'hybridation avec un polynucléotide à brin unique cible, le fragment chimioluminescent et le fragment absorbeur/émetteur sont suffisamment proches l'un de l'autre pour permettre un transfert non rayonnant d'énergie;

b) le contact ultérieur de l'échantillon de polynucléotide à brin unique avec des réactifs chimioluminescents efficaces pour provoquer une émission de lumière en présence du fragment chimioluminescent; et

c) la mesure de la quantité de lumière émise par le fragment absorbeur/émetteur.

2. Un procédé selon la revendication 1, dans lequel l'échantillon de polynucléotide à brin unique est mis en contact avec le premier et le second segments réactifs de polynucléotides avant d'être mis en contact avec les réactifs chimioluminescents.

3. Un procédé selon la revendication 1 ou la revendication 2, dans lequel le fragment chimioluminescent est choisi parmi une peroxydase et les luciférases.

4. Un procédé selon l'une quelconque des revendications précédentes dans lequel le fragment absorbeur/émetteur est choisi parmi les fluorophores et les lumenophores.

5. Un procédé selon l'une quelconque des revendications précédentes dans lequel le premier et le second segments réactifs de polynucléotides à brin unique sont obtenus par:

a) clivage de polynucléotides cibles à double brin dans un échantillon physiologique pour former des segments de polynucléotides;

b) dénaturation des segments de polynucléotides pour obtenir des segments de polynucléotides à brin unique;

c) marquage terminal des extrémités 3' des segments de polynucléotides avec soit un fragment chimioluminescent soit un fragment absorbeur/émetteur; et

d) marquage terminal des extrémités 5' des segments de polynucléotides avec le fragment non utilisé dans le stade (c).

6. Un procédé selon la revendication 5 dans lequel le fragment chimioluminescent est fixé aux extrémités 5' des segments réactifs de polynucléotides et le fragment absorbeur/émetteur est fixé aux extrémités 3' des segments réactifs de polynucléotides.

7. Un procédé selon la revendication 5 dans lequel le fragment chimioluminescent est fixé aux extrémités 3' des segments de polynucléotides et le fragment absorbeur/émetteur est fixé aux extrémités 5' des segments de polynucléotides.

8. Un réactif pour la détection de la présence d'un polynucléotide à brin unique cible dans un échantillon de polynucléotides à brin unique comprenant:

a) un premier segment réactif de polynucléotide à brin unique ayant au moins un fragment chimioluminescent qui lui est fixé et qui est complémentaire d'une portion d'un polynucléotide à brin unique cible;

b) un second segment réactif de polynucléotide à brin unique ayant au moins un fragment absorbeur/émetteur qui lui est fixé et qui est complémentaire d'une portion différente s'excluant mutuellement du polynucléotide à brin unique cible; et

c) des réactifs chimioluminescents efficaces pour provoquer une émission de lumière en présence du fragment chimioluminescent.

9. Un réactif selon la revendication 8 dans lequel un fragment chimioluminescent est fixé à une extrémité du premier segment réactif de polynucléotide à brin unique.

10. Un réactif selon la revendication 8 ou la revendication 9 dans lequel un fragment absorbeur/émetteur est fixé à une extrémité du second segment réactif de polynucléotide à brin unique.

11. Un réactif selon la revendication 8 dans lequel le premier segment réactif de polynucléotide à brin unique contient un fragment chimioluminescent et un fragment absorbeur/émetteur.

12. Un réactif selon la revendication 8 dans lequel le premier et le second segments réactifs de polynucléotides à brin unique contiennent tous deux un fragment chimioluminescent et un fragment absorbeur/émetteur.

13. Un réactif selon la revendication 12 dans lequel le fragment chimioluminescent et le fragment absorbeur/émetteur sont fixés aux extrémités du premier et du second segments réactifs de polynucléotides.

14. Un réactif selon la revendication 13 dans lequel le fragment chimioluminescent est fixé à l'extrémité 3' du premier et du second segments réactifs de polynucléotides à brin unique et un fragment absorbeur/émetteur est fixé à l'extrémité 5' du premier et du second segments réactifs de polynucléotides à brin unique.

15. Un réactif selon l'une quelconque des revendications 8 à 14 dans lequel le fragment chimioluminescent est choisi parmi une peroxydase et les luciférases.

16. Un réactif selon l'une quelconque des revendications 8 à 14 dans lequel le fragment absorbeur/émetteur est choisi parmi les fluorophores et les lumenophores.

17. Un procédé d'analyse d'une séquence de polynucléotide particulière ou d'un segment de gène particulier comprenant:

a) le contact d'un échantillon de polynucléotide à brin unique dans des conditions d'hybridation avec une solution réactive comprenant un premier et une second segments de polynucléotides à brin unique qui sont complémentaires de portions s'excluant mutuellement d'un polynucléotide à brin unique cible, ledit premiet et ledit second segments ayant tous deux un fragment absorbeur/émetteur différent qui leur est fixé, si bien que lors de l'hybridation avec un polynucléotide à brin unique cible, les fragments absorbeurs/émetteurs sont suffissamment proches l'un de l'autre pour permettre un transfer non rayonnant d'énergie;

b) l'irradiation de l'échantillon avec une lumière suffisante pour exciter un des fragments absorbeurs/émetteurs; et

c) la mesure de la réponse lumineuse de l'autre fragment absorbeur/émetteur.

18. Une solution réactive comprenant un premier et un second segments de polynucléotides à brin unique qui sont complémentaires de portions s'excluant mutuellement d'un polynucléotide à brin unique cible, ledit premiet et ledit second segments ayant tous deux un fragment absorbeur/émetteur différent qui leur est fixé, si bien que lors de l'hybridation avec un polynucléotide à brin unique cible, les fragments absorbeurs/émetteurs sont suffissamment proches l'un de l'autre pour permettre un transfert non rayonnant d'énergie.

**Patentansprüche**

1. Verfahren zur Prüfung einer einzigartigen Polynucleotidsequenz oder eines einzigartigen Gensegments, gekennzeichnet durch

a) Behandlung einer einsträngigen Polynucleotidprobe unter Hybridisierungsbedingungen mit einem ersten einsträngigen Polynucleotidreagenzsegment und einem zweiten einsträngigen Polynucleotidreagenzsegment, wobei diese Segmente komplementär sind zu sich gegenseitig ausschließenden Teilen eines als Ziel gewünschten einsträngigen Polynucleotids und wobei an das erste Reagenzsegment ein Chemillumineszenzrest und an das zweite Reagenzsegment ein Absorber/Emitter-Rest jeweils derart gebunden ist, daß nach Hybridisierung mit einem das gewünschte Ziel bildenden einsträngigen Polynucleotid der Chemilumineszenzrest und der Absorber/Emitter-Rest so eng aneinander liegen, daß eine nicht strahlende Energieübertragung möglich ist,

b) Weiterbehandlung der einsträngigen Polynucleotidprobe mit Chemilumineszenzreagenzien, die in Gegenwart des Chemilumineszenzrests eine Lichtemission hervorrufen, und

c) Messung der Menge an von Absorber/Emitter-Rest emittiertem Licht.

2. Verfahren nach Anspruch 1, dadurch gekennzeichnet, daß man die einsträngige Polynucleotidprobe mit dem ersten Polynucleotidreagenzsegment und dem zweiten Polynucleotidreagenzsegment behandelt, bevor man die Behandlung mit den Chemilumineszenzreagenzien durchführt.

3. Verfahren nach Anspruch 1 oder 2, dadurch gekennzeichnet, daß der Chemilumineszenzrest ausgewählt ist aus Peroxidasen und Lucierasen.

4. Verfahren nach irgendeinem der vorhergehenden Ansprüche, dadurch gekennzeichnet, daß der Absorber/Emitter-Rest ausgewählt ist aus Fluorophoren und Phosphoren.

5. Verfahren nach irgendeinem der vorhergehenden Ansprüche, dadurch gekennzeichnet, daß die erste einsträngige Polynucleotidreagenzsequenz und die zweite einsträngige Polynucleotidreagenzsequenz erhalten worden sind durch

a) Spaltung doppelsträngiger Zielpolynucleotide in einer physiologischen Probe unter Bildung von Polynucleotidsegmenten,

b) Denaturierung der Polynucleotidsegmente unter Bildung einsträngiger Polynucleotidsegmente,

c) Endmarkierung der Enden 3' der Polynucleotidsegmente mit entweder einem Chemilumineszenz-rest oder einem Absorber/Emitter-Rest und

d) Endmarkierung der Enden 5' der Polynucleotidsegmente mit dem in obiger Stufe c) jeweils nicht verwendeten Rest.

6. Verfahren nach Anspruch 5, dadurch gekennzeichnet, daß der Chemilumineszenzrest an die Enden 5' der Polynucleotidreagenzsegmente und der Absorber/Emitter-Rest an die Enden 3' der Polynucleotidreagenzsegmente gebunden wird.

7. Verfahren nach Anspruch 5, dadurch gekennzeichnet, daß der Chemilumineszenzrest an die Enden 3' der Polynucleotidsegmente und der Absorber/Emitter-Rest an die Enden 5' der Polynucleotidsegmente gebunden wird.

8. Reagenz zur Detektion der Anwesenheit eines das gewünschte Ziel bildenden einsträngigen Polynucleotids in einer einsträngigen Polynucleotidprobe, gekennzeichnet durch

a) ein erstes einsträngiges Polynucleotidreagenzsegment, an das wenigstens ein Chemilumineszenzrest gebunden ist und das zu einem Teil eines das gewünschte Ziel bildenden einsträngigen Polynucleotids komplementär ist,

b) ein zweites einsträngiges Polynucleotidreagenzsegment, an das wenigstens ein Absorber/Emitter-Rest gebunden ist und das zu einem anderen, sich gegenseitig ausschließenden Teil des das gewünschte Ziel bildenden einsträngigen Polynucleotids komplementär ist, und

c) Chemilumineszenzreagenzien, die in Gegenwart des Chemilumineszenzrests eine Lichtemission hervorrufen.

9. Reagenz nach Anspruch 8, dadurch gekennzeichnet, daß ein Chemilumineszenzrest an ein Ende des ersten einsträngigen Polynucleotidreagenzsegments gebunden ist.

10. Reagenz nach Anspruch 8 oder 9, dadurch gekennzeichnet, daß ein Absorber/Emitter-Rest an ein Ende des zweiten einsträngigen Polynucleotidreagenzsegments gebunden ist.

11. Reagenz nach Anspruch 8, dadurch gekennzeichnet, daß das erste einsträngige Polynucleotidreagenzsegment einen Chemilumineszenzrest und einen Absorber/Emitter-Rest enthält.

12. Reagenz nach Anspruch 8, dadurch gekennzeichnet, daß sowohl das erste als auch das zweite einsträngige Polynucleotidreagenzsegment einen Chemilumineszenzrest und einen Absorber/Emitter-Rest enthält.

13. Reagenz nach Anspruch 12, dadurch gekennzeichnet, daß der Chemilumineszenzrest und der Absorber/Emitter-Rest an die Enden von sowohl dem ersten als auch dem zweiten Polynucleotidreagenzsegment gebunden sind.

14. Reagenz nach Anspruch 13, dadurch gekennzeichnet, daß der Chemilumineszenzrest an das Ende 3' des ersten und des zweiten einsträngigen Polynucleotidreagenzsegments gebunden ist, und daß ein Absorber/Emitter-Rest an das Ende 5' des ersten und des zweiten einsträngigen Polynucleotidreagenzsegments gebunden ist.

15. Reagenz nach irgendeinem der Ansprüche 8 bis 14, dadurch gekennzeichnet, daß der Chemilumineszenzrest ausgewählt ist aus Peroxidasen und Luciferasen.

16. Reagenz nach einem der Ansprüche 8 bis 14, dadurch gekennzeichnet, daß der Absorber/ Emitter-Rest ausgewählt ist aus Fluorophoren und Phosphoren.

17. Verfahren zur Prüfung einer einzigartigen Polynucleotidsequenz oder eines einzigartigen Gensegments, gekennzeichnet durch

a) Behandlung einer einsträngigen Poly- nucleotidprobe unter Hybridisierungsbeding- ungen mit einer Reagenzlösung aus einem ersten einsträngigen Polynucleotidsegment und einem zweiten einsträngigen Polynucleotid- reagenzsegment, wobei diese Segmente kom- plementär sind zu sich gegenseitig ausschließ enden Teilen eines als Ziel gewünschten einsträngigen Polynucleotids und wobei an das erste Segment und das zweite Segment ein jeweils unterschiedlicher Absorber/Emitter-Rest derart gebunden ist, daß nach Hybridiserung mit einem das gewünschte Zeil bildenden einsträngigen Polynucleotid die Absorber/Emitter-Reste so eng aneinander liegen, daß eine nicht strahlende Energieübertragung möglich ist,

b) Bestrahlung der Probe mit soviel Licht, daß einer der Absorber/Emitter-Reste angeregt wird, und

c) Messung des Lichtsignals des anderen Absorber/Emitter-Restes.

18. Reagenzlösung, gekennzeichnet durch einer Gehalt an einem ersten einsträngigen Polynucleotidsegment und einem zweiten einsträngigen Polynucleotidsegment, wobei diese Segmente komplementär sind zu sich gegenseitig ausschließenden Teilen eines als Zeil gewünschten einsträngigen Polynucleotids und wobei sowohl an das erste Segment als auch das zweite Segment ein jeweils unterschiedlicher Absorber/ Emitter-est derart gebunden ist, daß nach Hybridisierung mit einem das gewünschte Ziel bildenden einsträngigen Polynucleotid die Absorber/Emitter-Reste so eng aneinander liegen, daß eine nicht strahlende Energieübertragung möglich ist.

**Fig. 1.** PREPARATION OF SINGLE-STRANDED POLYNUCLEOTIDE REAGENT SEGMENTS FOR ASSAYING ANTIBIOTIC RESISTANCE

① ISOLATION AND RESTRICTION OF DNA OF INTEREST

BACTERIAL PLASMID

RESISTANCE GENE

RESTRICTION ENDONUCLEASE

② ISOLATION OF THE RESISTANCE GENE

+

RESISTANCE GENE

③ CLEAVAGE OF RESISTANCE GENE INTO TWO PIECES X AND Y

RESISTANCE GENE

3'————————5'
5'————————3'

RESTRICTION ENDONUCLEASE

X(+)
3'————5'
5'————3'
X(−)

+

Y(+)
3'————5'
5'————3'
Y(−)

④ ISOLATION OF (+) STRANDS

DENATURE
REMOVE X(−) AND Y(−) STRAND

3'———— X(+) ————5'  +  3'———— Y(+) ————5'

⑤ END LABELING WITH CHEMILUMINESCENT AND ABSORBER/ EMITTER MOIETIES

(CL)—

(A/E)—

3'———— X(+) ————5'(CL)

(A/E) 3'———— Y(+) ————5'

5'-CHEMILUMINESCENT LABELED X(+) STRAND

3'-ABSORBER/EMITTER LABELED Y(+) STRAND

# fig.2.

(1) ISOLATION OF DNA FROM SAMPLE

←ANTIBIOTIC RESISTANCE GENE

(3) ADDITION OF SINGLE-STRANDED POLYNUCLEOTIDE REAGENT SEGMENTS AND HYBRIDIZATION

(2) DENATURATION

$X(+)$ 3'|||||||||| 5' (CL)

$Y(+)$ (AE) 3'|||||||||| 5'

SINGLE STRANDS OF SAMPLE DNA

ANTIBIOTIC RESISTANCE (TARGET) STRANDS

$Y(-)$  $Y(+)$ (AE) (CL)

$X(-)$  $X(+)$

(4) HYBRIDIZATION OCCURS BETWEEN REAGENT STRANDS AND COMPLIMENTARY STRAND FROM SAMPLE PROXIMATING THE CHEMILUMINESCENT AND ABSORBER/EMITTER MOIETIES.

INDUCED EMISSION

(AE)

(CL)

(5) DETECTION OF INDUCED FLUORESCENCE

CHEMILUMINESCENCE

PMT TUBE

CUT OFF FILTER